# EUROPEAN PATENT APPLICATION

(11) **EP 3 824 922 A1**
(43) Date of publication of application: **26.05.2021**
(21) Application number: 20207922.4
(22) Date of filing: 16.11.2020
(51) Int. Cl.: A61M 5/00, A61B 17/34

(54) **EXTERNAL AND INTERNAL EXPANSION VIBRATION LIPOFILLING SYSTEMS**

(30) Priority: 19.11.2019 US 201962937590 P
(71) Applicant: Surgistem Technologies, LLC, Boston, MA 02116 (US)
(72) Inventor: DEL VECCHIO, Daniel A., Boston, MA Massachusetts 02116 (US); WALL Jr., Simeon, Shreveport, LA Louisiana 71105 (US)
(74) Representative: Appleyard Lees IP LLP

(57) **Abstract**

External and internal expansion vibration lipofilling methods for shaping the skin of a patient may include modifying the body surface of a patient by forming a tissue injection space in the skin of the patient by externally applying negative pressure to the skin of the patient and simultaneously injecting adipose cells into the tissue injection space. External and internal expansion vibration lipofilling systems for shaping the skin of a patient are also disclosed.

## Description

### FIELD

Illustrative embodiments of the disclosure relate to lipofilling methods, such as non-therapeutic (for example cosmetic) lipofilling methods. More particularly, illustrative embodiments of the disclosure relate to external and internal expansion vibration lipofilling systems and methods in which a subdermal tissue injection space may be created by external application of negative pressure and internal application of positive pressure to a patient's skin to increase the volume and enhance targeted placement of adipose tissue injected into the skin during a lipofilling procedure.

### SUMMARY

Illustrative embodiments of the disclosure are generally directed to external and internal expansion vibration lipofilling methods for shaping the body surface of a patient. An illustrative embodiment of the methods may include modifying the body surface of the patent by forming a tissue injection space in the skin of the patient by externally applying negative pressure to the skin and simultaneously injecting adipose cells into the tissue injection space.

In some embodiments, the methods may further include forming the tissue injection space by internally applying positive pressure by vibration of the cannula of a tissue injection device as the adipose cells are injected through the cannula into the tissue injection space.

Illustrative embodiments of the disclosure are further generally directed to external and internal expansion vibration lipofilling systems. An illustrative embodiment of the systems may include a medical suction apparatus having an air pump and a suction device disposed in pneumatic communication with the air pump. A tissue injection device may be configured to be used in conjunction with the medical suction apparatus. The tissue injection device may include a gripping portion. A cannula may be disposed in fluid communication with the gripping portion. A pump may be disposed in fluid communication with the cannula. A container may be disposed in fluid communication with the pump. The suction device of the medical suction apparatus may be configured to induce formation of a tissue injection space in the skin of the patient by externally applying negative pressure to the skin of the patient. The tissue injection device may be operable to simultaneously inject adipose cells from the container through the gripping portion and the cannula, respectively, into the tissue injection space.

In some applications, the tissue injection space may be additionally formed by internally applying positive pressure by vibrating the cannula of the tissue injection device as the adipose cells are injected through the cannula into the tissue injection space.

The present invention provides an external and internal expansion vibration lipofilling system for shaping the body surface of a patient, comprising:
a medical suction apparatus having an air pump and a suction device (20) disposed in pneumatic communication with the air pump; and
a tissue injection device configured to be used in conjunction with the medical suction apparatus, the tissue injection device including:
   a gripping portion;
   a cannula disposed in fluid communication with the gripping portion;
   a pump disposed in fluid communication with the cannula; and
   the suction device of the medical suction apparatus configured to induce formation of a tissue injection space in the skin of the patient by applying negative pressure to the skin of the patient; and
   the tissue injection device operable to simultaneously inject adipose cells from the container through the gripping portion and the cannula, respectively, into the tissue injection space.

The system may further comprise a vibratory motor engaging the gripping portion for vibration, preferably wherein the vibratory motor is electric, hydraulic, pneumatic or any combination thereof.

The present invention further provides an external and internal expansion vibration lipofilling method for shaping the body surface of a patient, comprising:
modifying the body surface of the patient by:
forming a tissue injection space in the skin of the patient by externally applying negative pressure to the skin of the patient; and
simultaneously injecting adipose cells into the tissue injection space.

The method may further comprise vibrating the adipose cells simultaneous with injecting the adipose cells into the tissue injection space. Vibrating the adipose cells may comprise vibrating the adipose cells with a frequency within the range of about 2,000-10,000 cycles/min, preferably wherein vibrating the adipose cells within the range of about 2,000-10,000 cycles/min. comprises vibrating the adipose cells with a frequency of about 7,000 cycles/min.

The method may further comprise forming an incision in the skin, and wherein simultaneously injecting the adipose cells into the tissue injection space comprises simultaneously injecting the adipose cells into the tissue injection space through the incision.

Forming a tissue injection space in the skin of the patient by externally applying negative pressure to the skin of the patient may comprise forming a raised or convex suctioned skin area on the surface of the skin over the tissue injection space.

Modifying the body surface of the patient may comprise eliminating at least one skin crease in the skin or may comprise augmenting a breast on the patient.

The method may further comprise migrating the adipose cells into subdermal regions of the skin.

The present invention may further provide an external and internal expansion vibration lipofilling method for shaping the body surface of a patient, comprising:
modifying the body surface of the patient by:
obtaining an external and internal expansion vibration lipofilling system including a tissue injection device having a cannula with a cannula tip and a medical suction apparatus having a suction device;
forming an incision in the skin adjacent to a recipient injection site;
placing the suction device of the medical suction apparatus against the surface of the skin over the recipient injection site to achieve an airtight seal between the suction device and the skin;
forming a tissue injection space in the ski of the patient at the recipient injection site by externally applying negative pressure to the skin of the patient through the suction device at the recipient injection site;
inserting the cannula of the tissue injection device through the incision until the cannula tip of the cannula terminates in the tissue injection space; and
injecting adipose cells through the cannula into the tissue injection space while maintaining the suction device in place on the skin.

The method may further comprise vibrating the cannula of the tissue injection device simultaneous with injecting the adipose cells into the tissue injection space. Vibrating the cannula of the tissue injection device may comprise vibrating the cannula of the tissue injection device with a frequency within the range of about 2,000-10,000 cycles/min, preferably wherein vibrating the cannula of the tissue injection device with the frequency within the range of about 2,000-10,000 cycles/min. comprises vibrating the cannula of the tissue injection device with a frequency of about 7,000 cycles/min.

Forming the tissue injection space in the skin of the patient at the recipient injection site by externally applying negative pressure to the skin of the patient through the suction device at the recipient injection site may comprise forming a raised or convex suctioned skin area on the surface of the skin over the tissue injection space.

Modifying the body surface of the patient may comprise eliminating at least one skin crease in the skin, or may comprise augmenting a breast on the patient.

The method may further comprise migrating the adipose cells into subdermal regions of the skin by gliding the suction device on the medical suction apparatus over the skin.

The system and methods of the present invention provide non-therapeutic (for example cosmetic) effects to the patient. In other words, the method of the present invention is a non-therapeutic, i.e. a cosmetic, method. The system and method act to improve the bodily appearance of a patient. Thus, references herein to shaping or modifying the body surface of a patient are to shaping or modifying so as to improve the bodily appearance of the patient.

The present invention also provides the use of a system according to the present invention to form a tissue injection space in the skin of a patient by externally applying negative pressure to the skin of the patient; and to simultaneously inject (and, for example, simultaneously vibrate) adipose cells into the tissue injection space.

The present invention also provides the use of a system according to the present invention to achieve an airtight seal between the suction device and the skin, to form a tissue injection space in the skin of a patient by externally applying negative pressure to the skin of the patient; and to simultaneously inject (and, for example, simultaneously vibrate) adipose cells into the tissue injection space.

The present invention also provides the use of a system according to the present invention to shape the body surface of a patient, for example by eliminating at least one skin crease in the skin.

The present invention also provides the use of a system according to the present invention to modify the body surface of a patient, for example by eliminating at least one skin crease in the skin.

The present invention also provides the use of a system according to the present invention to shape the body surface of a patient, for example by augmenting a breast on the patient.

The present invention also provides the use of a system according to the present invention to modify the body surface of a patient, for example by augmenting a breast on the patient.

### BRIEF DESCRIPTION OF THE DRAWINGS

Illustrative embodiments of the disclosure will now be described, by way of example, with reference to the accompanying drawings, in which:
FIG. 1 is a block diagram of an illustrative embodiment of an external and internal vibration lipofilling system which is suitable for implementation of the external and internal expansion vibration lipofilling methods of the disclosure;
FIG. 2 is a perspective view, partially in section, of a patient, with a suction device of the system placed against the skin over a recipient injection site on the patient and a cannula on a tissue injection device of the system inserted through an incision in the skin in typical implementation of the external and internal expansion vibration lipofilling methods;
FIG. 3 is a sectional view of the suction device, placed against the skin of the patient over the recipient injection site, more particularly illustrating external application of negative pressure to the skin through the suction device and internal application of positive pressure inside the skin as the cannula of the tissue injection device is vibrated or reciprocated in the subdermal space beneath the recipient injection site;
FIG. 4 is a cross-sectional view of the patient's skin with the suction device (illustrated in section) placed against the skin over a pair of skin creases at the recipient injection site preparatory to external application of negative pressure to the skin through the suction device;
FIG. 5 is a cross-sectional view of the patient's skin, more particularly illustrating external application of negative pressure to the skin through the suction device and formation of a suctioned skin area on the skin at the recipient injection site beneath the suction device and a subdermal tissue injection space beneath the suctioned skin area;
FIG. 6 is a cross-sectional view of the patient's skin with the suction device remaining in place and externally applying negative pressure to the surface of the skin as a cannula of a tissue injection device is inserted through an incision in the skin into the tissue injection space;
FIG. 7 is a cross-sectional view of the patient's skin illustrating typical initial injection of adipose cells through the cannula of the tissue injection device into the tissue injection space as the suction device continues to externally apply negative pressure to the surface of the skin and the vibrating cannula internally applies positive pressure inside the skin to augment dispersal or enhance homogenous distribution of the adipose cells throughout the tissue injection space;
FIG. 8 is a cross-sectional view of the patient's skin illustrating completion of the injection of the adipose cells through the cannula into the tissue injection space as external application of the negative air pressure to the skin through the suction device and internal application of positive pressure inside the skin through the vibrating cannula is maintained;
FIG. 9 is a cross-sectional view of the patient's skin after removal of the suction device from the skin and removal of the cannula from the incision;
FIGS. 10-13 are cross-sectional views of a patient's skin after typical injection of the adipose cells into the tissue injection space, more particularly illustrating sequential gliding movement of the suction device over the skin to migrate adipose cells into the subdermal regions of the skin;
FIG. 14 is a flow diagram of an illustrative embodiment of the external and internal expansion vibration lipofilling methods;
FIG. 15 is a flow diagram of a typical method of facilitating migration of the adipose cells into subdermal regions of skin after injection of the adipose cells into the skin according to some embodiments of the external and internal expansion vibration lipofilling methods; and
FIGS. 16-18 are cross-sectional views of a female breast illustrating typical implementation of the external and internal vibration lipofilling methods in a breast augmentation procedure.

### DETAILED DESCRIPTION

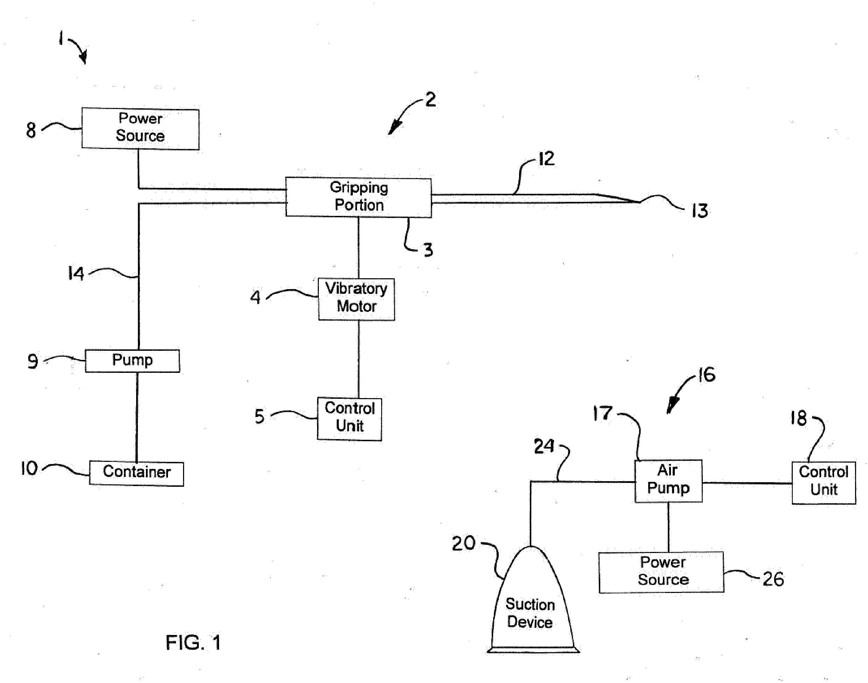

The following detailed description is merely exemplary in nature and is not intended to limit the described embodiments or the application and uses of the described embodiments. As used herein, the word "exemplary" or "illustrative" means "serving as an example, instance, or illustration." Any implementation described herein as "exemplary" or "illustrative" is not necessarily to be construed as preferred or advantageous over other implementations. All of the implementations described below are exemplary implementations provided to enable persons skilled in the art to make or use the embodiments of the disclosure and are not intended to limit the scope of the disclosure, which is defined by the claims. For purposes of description herein, the terms "upper", "lower", "left", "rear", "right", "front", "vertical", "horizontal", and derivatives thereof shall relate to the invention as oriented in FIG. 1. Furthermore, there is no intention to be bound by any expressed or implied theory presented in the preceding technical field, background, brief summary or the following detailed description. It is also to be understood that the specific devices and processes illustrated in the attached drawings, and described in the following specification, are simply exemplary embodiments of the inventive concepts defined in the appended claims. Hence, specific dimensions and other physical characteristics relating to the embodiments disclosed herein are not to be considered as limiting, unless the claims expressly state otherwise.

Referring initially to FIGS. 1-3 of the drawings, an illustrative embodiment of the external and internal expansion vibration lipofilling systems, hereinafter system, of the disclosure which is suitable for implementation of the external and internal expansion vibration lipofilling methods, hereinafter method, of the disclosure is generally indicated by reference numeral 1 in FIG. 1. The system 1 may include a tissue injection device 2. For example and without limitation, in some applications, the tissue injection device 2 may have a design which is the same as or similar to that which is disclosed in U.S Pub. No. 2013/0310749, which is hereby incorporated by reference herein in its entirety. The tissue injection device 2 may include a gripping portion 3. In some applications, at least one vibratory motor 4 may engage the gripping portion 3 for vibration. The vibratory motor 4 may be electric, hydraulic and/or pneumatic. For example and without limitation, in some applications, the vibratory motor 4 may have a design which is the same as or similar to those described with respect to the electric vibrational apparatuses or the pneumatic vibrational apparatuses which are disclosed in U.S. Pat. No. 10,173,000, which patent is hereby incorporated by reference herein in its entirety.

A control unit 5 may operationally interface with the vibratory motor 4. The control unit 5 may include but is not limited to buttons, switches, slides, triggers, touch screens, voice recognition and/or other input control devices which are operable to control various operational parameters such as the vibration frequency and/or the vibration amplitude, for example and without limitation, of the vibratory motor 4 according to the knowledge of those skilled in the art. The control unit 5 may additionally include a display which indicates such data as status information and/or vibrational intensity (rate and/or amplitude), for example and without limitation.

At least one power source 8 may operably interface with the gripping portion 3. The power source 8 may include at least one battery and/or at least one electrical wall outlet, for example and without limitation.

An elongated cannula 12 may extend from the gripping portion 3. The cannula 12 may be coupled to the gripping portion 3 using a locked thread connector such as a Luer lock connector, for example and without limitation. In some embodiments of the tissue injection device 2, the vibratory motor 4 may engage the cannula 12 for oscillation or reciprocation directly or indirectly through the gripping portion 3. The cannula 12 may include a trocar or any other type of needle which is typically used for fat or tissue injection procedures or the like and may have a sharpened, pointed or tapered cannula tip 13. In some applications, the cannula 12 may include an exploded-tip or expanded basket cannula such as those which are described in U.S. Pat. No. 10,188,280, which patent is hereby incorporated by reference herein in its entirety. A non-limiting example of a cannula 12 which is suitable for implementation of the method is a 4-5 mm exploded tip cannula available from Surgistem Technologies of Boston, MA. In some applications, the cannula 12 may be angled to facilitate farther "reach" of the cannula 12 during the lipo-filling procedure, resulting in more extensive and thorough equalization of recipient-site tissue. More thorough and dispersed fat may translate to more surface area contact between grafted fat lobules and recipient tissue.

A pump 9 may be disposed in fluid communication with the gripping portion 3 such as through a suitable tubing 14. At least one canister or container 10 may be disposed in fluid communication with the pump 9. The pump 9 may include a peristaltic pump or any type of positive displacement pump or other automated and/or manual mechanical delivery system which is known by those skilled in the art and operable to pump a liquid, semisolid and/or solid material from the container 10 through the gripping portion 3 and the cannula 12, respectively. In some applications, the pump 9 may be configured to operate in both a suction (negative pressure) phase and an injection (positive pressure) phase. For example and without limitation, in some applications, the pump 9 may include a syringe such as a Toomey syringe known by those skilled in the art. In some embodiments, the pump 9 and the container 10 may be combined in the same device or apparatus.

As further illustrated in FIG. 1, the system 1 may further include a medical suction apparatus 16. The medical suction apparatus 16 may include an air pump 17. At least one power source 26 may operably interface with the air pump 17. The power source 26 may include at least one battery and/or at least one electrical wall outlet, for example and without limitation.

A control unit 18 may operationally interface with the air pump 17. The control unit 18 may be operable to control various operational parameters such as the suction strength and the suction and injection phases, for example and without limitation, of the air pump 17 according to the knowledge of those skilled in the art.

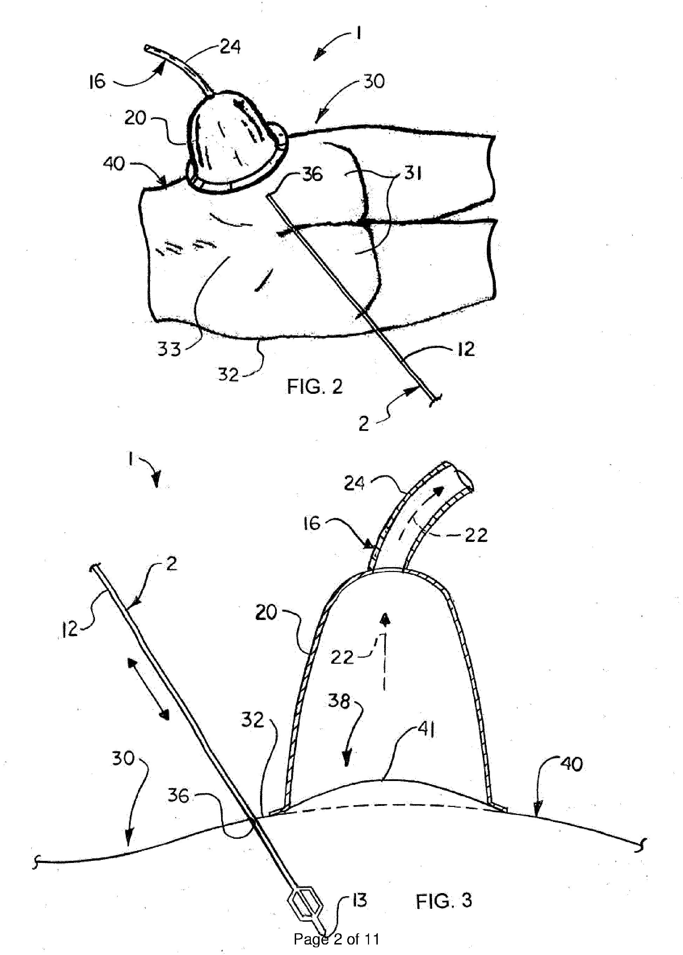

A suction device 20 may be disposed in pneumatic communication with the air pump 17 typically through suitable tubing 24. The suction device 20 may include a cup, dome or other concave structure which can be placed against a surface to impart a substantially airtight seal against the surface. Accordingly, in typical application of the system 1, as illustrated in FIG. 3 and will be hereinafter further described, the suction device 20 may be placed against the skin 40 of a patient 30 to exert negative pressure 22 against the skin 40 responsive to operation of the air pump 17. The tubing 24 may be flexible to facilitate selective movement and placement of the suction device 20 on or with respect to the patient 30.

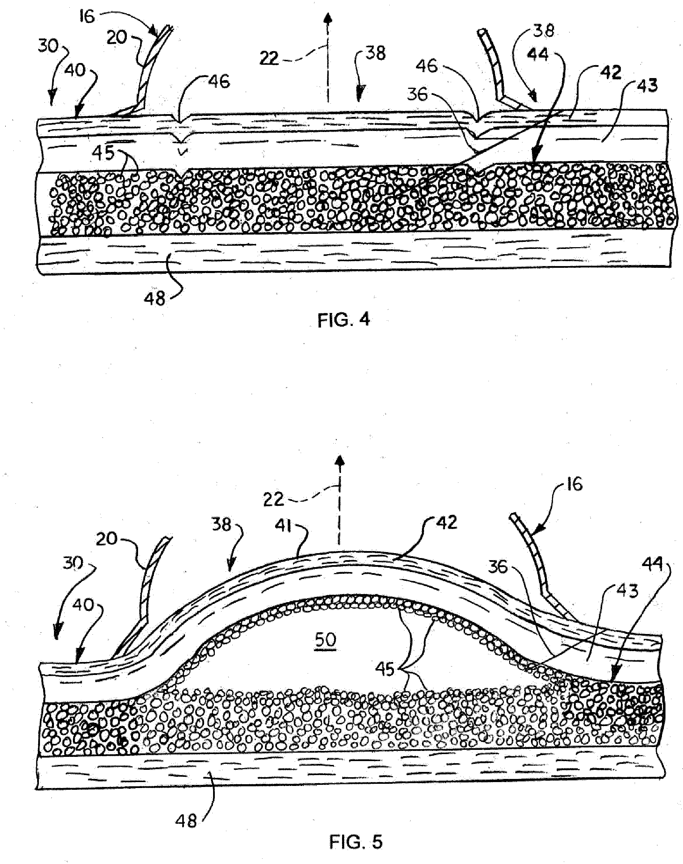

Referring next to FIGS. 2-9 of the drawings, in typical implementation of the external and internal expansion vibration lipofilling methods, the system 1 (FIG. 1) may be used to modify the body surface of a patient 30 by volumetrically enhancing, augmenting or filling out and smoothening the contour of the skin 40 of the patient 30, such as via percutaneous injection or injection directly into subcutaneous fat, as part of a fat transplantation procedure. For example and without limitation, the method may be implemented to rectify contour irregularities in the buttocks 31, hip 32 and/or lower back 33 of the patient 30. As illustrated in FIG. 4, the skin 40 of the patient 30 has an epidermis 42 and an underlying dermis 43. An adipose tissue layer 44 having adipose cells 45 underlies the subdermal space beneath the dermis 43. A muscle layer 48 underlies the adipose tissue layer 44. In some embodiments of the methods, the system 1 may be used in a breast augmentation procedure.

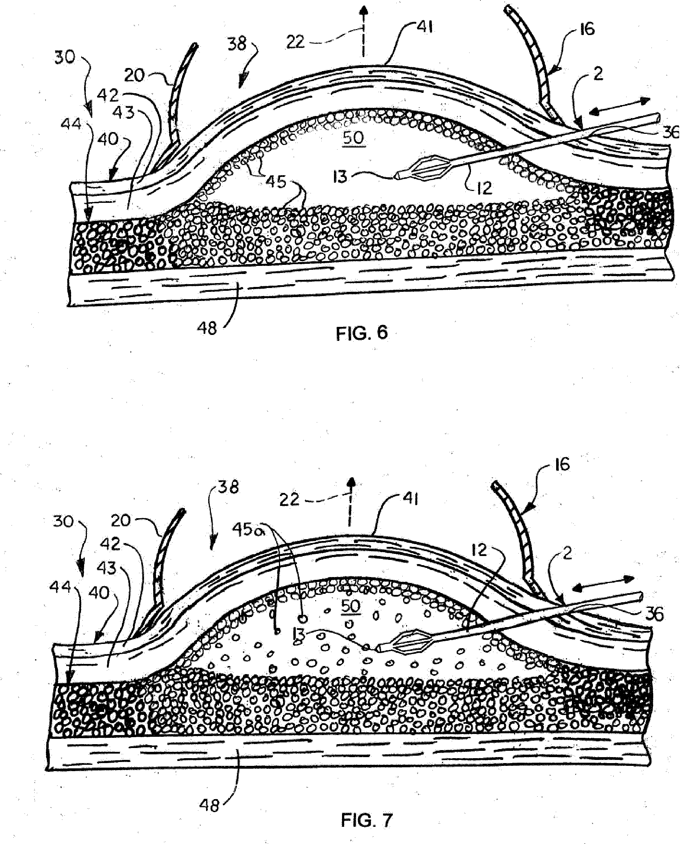

In the non-limiting example illustrated in FIGS. 4-9, the method may be implemented in a lipo-injection procedure to eliminate a pair of skin creases 46 (FIG. 4) at a recipient injection site 38 (FIG. 3) in the skin 40 as well as volumetrically enhance, augment or fill out the contour of the skin 40 at the recipient injection site 38. Accordingly, in some implementations of the method, after donor tumescence, a sufficient volume of adipose cells 45a (FIG. 7) which will subsequently be injected into the skin 40 at the recipient injection site 38 may initially be harvested from at least one harvest or donor site (not illustrated) on the patient 30 typically by operation of the tissue injection device 2 (FIG. 1). Responsive to operation of the pump 9 in the suction phase, the harvested adipose cells 45a may be drawn from the donor site through the cannula 12, gripping portion 3 and tubing 14, respectively, into the container 10, after which the cannula 12 may be withdrawn from the donor site. In other implementations, the adipose tissue cells 45a which are to be injected at the recipient injection site 38 may be obtained from the donor site using a separate device.

As the patient typically lies in a supine or lateral position, an incision 36 may be formed in the skin 40 adjacent to the recipient injection site 38, as illustrated in FIGS. 2-4. As illustrated in FIG. 4, the incision 36 may extend through the epidermis 42 and the dermis 43 and into the underlying adipose tissue layer 44.

As illustrated in FIGS. 2-5, the suction device 20 on the medical suction apparatus 16 of the system 1 (FIG. 1) may be placed against the surface of the skin 40 over the recipient injection site 38 to achieve an airtight seal between the suction device 20 and the skin 40. The air pump 17 (FIG. 1) of the medical suction apparatus 16 may be operated to externally apply negative pressure 22 to the surface of the skin 40 through the tubing 24 and the interior of the suction device 20. As illustrated in FIGS. 3 and 5, the negative pressure 22 may form a raised or convex suctioned skin area 41 which substantially conforms to the size and shape of the suction device 20 on the surface of the skin 40 at the recipient injection site 38. The suctioned skin area 41 may include the epidermis 42 and the dermis 43 and may further include a portion of the adipose layer 44. As further illustrated in FIG. 5, a tissue injection space 50 may be formed in the adipose tissue layer 44 beneath the suctioned skin area 41.

As illustrated in FIGS. 2, 3 and 6, as the suction device 20 is maintained in place on the skin 40, the cannula 12 may be inserted through the incision 36 until the cannula tip 13 of the cannula 12 terminates in the tissue injection space 50. The pump 9 (FIG. 1) of the tissue injection device 2 may be operated in the injection phase to pump the harvested adipose cells 45a from the container 10 through the tubing 14, gripping portion 2 and cannula 12, respectively, and into the tissue injection site 50. A typical flow rate of the adipose cells 45a is 200-300 cc/min. In areas of excessive ligamentous bands, such as the midlateral and inferolateral buttock, flow of adipose tissue may be reduced or halted temporarily while the exploded-tip cannula 12 is manipulated to loosen or release the bands and diminish resistance in tight areas.

In some applications, the vibratory motor 4 (FIG. 1) of the tissue injection device 2 may simultaneously be operated to vibrate or reciprocate the injection cannula 12 and achieve enhanced dispersal and homogenous distribution of the harvested adipose cells 45a throughout the tissue injection site 50. The various operational parameters of the vibratory motor 4, such as the vibration frequency and/or the vibration amplitude, for example and without limitation, may be selected by input from the control unit 5. A typical vibration or reciprocation frequency for the injection cannula 12 may fall within the range of about 2,000-10,000 cycles/min, with a typical frequency of about 7,000 cycles/min.

In some applications, the expanded basket cannula 12 may be used in conjunction with the vibratory motion of the cannula 12 to facilitate internal positive expansion of the tissue injection site 50 in conjunction with the external negative expansion which is applied by the negative pressure 22 on the surface of the skin 40. Accordingly, the expanded basket cannula 12 may create differential high- and low-pressure zones in the tissue injection space 50 such that the adipose cells 45 migrate from the high-pressure zones to the low-pressure zones. Consequently, the internal positive pressure applied by the vibrating cannula 12, in conjunction with the external negative pressure applied by the suction device 20, may synergistically increase the volume of the tissue injection space 50 to increase the volume of adipose cells 45a which can be injected into the skin 40.

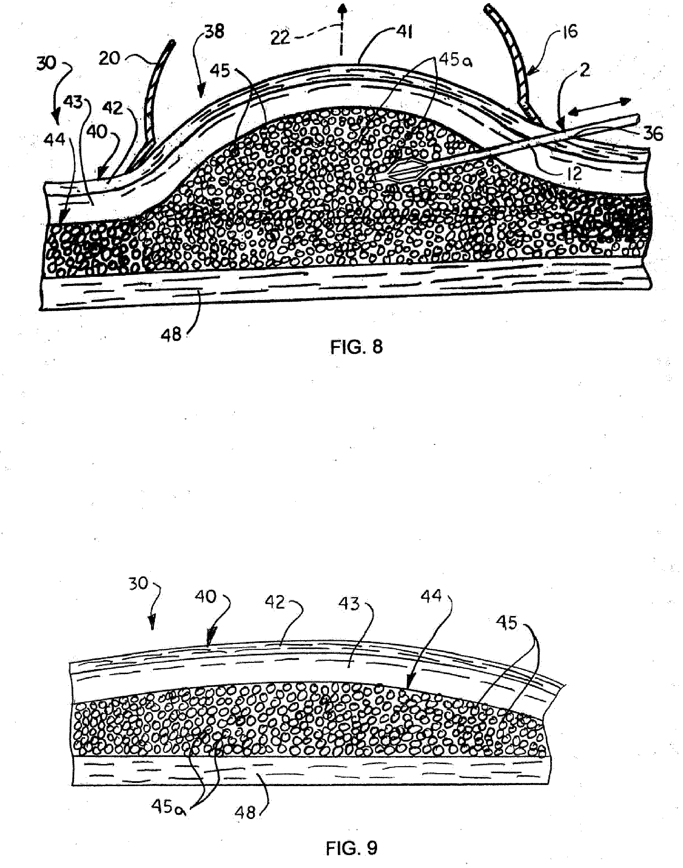

As illustrated in FIG. 8, injection of the harvested adipose cells 45a into the tissue injection space 50 may continue until the adipose tissue layer 44 contains the desired volume of adipose cells 45 to achieve the selected aesthetic contour of the skin 40 at the recipient injection site 38. Upon conclusion of the injection procedure, the injection cannula 12 may be removed from the incision 36, the suction device 20 removed from the skin 40 and the incision 36 closed. As illustrated in FIG. 9, after recovery, the treated skin 40 has a reshaped contour and lacks the skin creases 46 (FIG. 4), enhancing the aesthetic appearance of the skin 40 to the satisfaction of the patient 30.

It will be appreciated by those skilled in the art that the external and internal expansion vibration lipofilling methods and system of the disclosure may enable a surgeon to more easily place adipose cells in an enhanced targeted manner in the skin of the patient as compared to conventional lipofilling methods. The methods and system may facilitate placement of a larger number or volume of adipose cells into the patient's skin than can be achieved using conventional lipo-filling methods. The methods and system may enhance safety in the injection of adipose cells into the skin by enabling the surgeon to place the adipose cells more superficially in the adipose tissue layer, thereby preventing surgical misadventure into and inadvertent damage to the deeper underlying muscle layer and other anatomical structures. The adipose cells can be placed in the adipose tissue layer in a more targeted fashion, thereby obtaining better contour and projection due to the internal and external expansion of the tissue.

It will be further appreciated by those skilled in the art that the external and internal expansion vibration lipofilling methods and system of the disclosure may enable the surgeon to shift the adipose tissue cells more easily using the tissue injection device after injection. Shifting of cells may be carried out safely by equalizing the adipose cells more superficially in the adipose tissue layer, preventing damage to the underlying muscles and deeper anatomical structures. The adipose cells may be shifted more easily and in a more targeted fashion after injection to obtain enhanced contour and projection due to both internal and external expansion of the tissue.

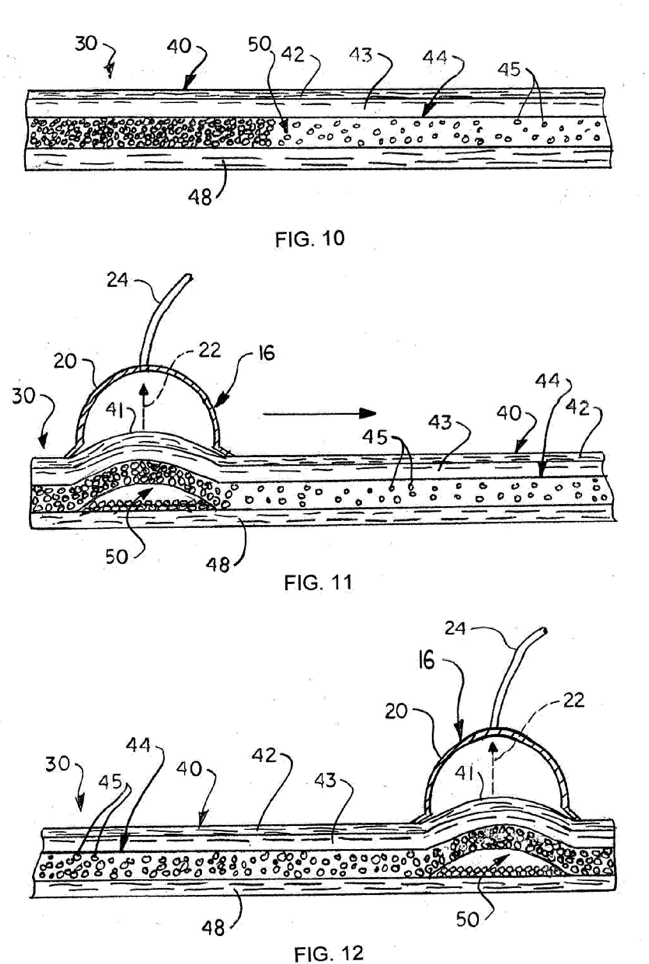

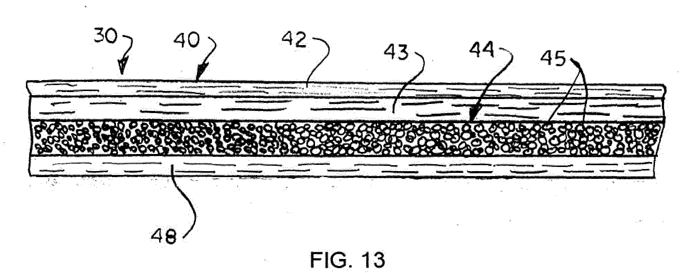

Referring next to FIGS. 10-13 of the drawings, in some applications, the medical suction apparatus 16 of the system 1 may be used to facilitate migration of adipose cells 45 into subdermal regions of the patient's skin 40 typically after typical injection of the adipose cells 45 into the tissue injection space 50, as was heretofore described with respect to FIGS. 2-9. Accordingly, the suction device 20 may initially be placed against the skin 40 of the patient 30 typically in the region or area of the recipient injection site 38. As the air pump 17 is operated to induce the negative pressure 22 in the suction device 20, the suction device 20 may simultaneously be moved in a gliding motion over the skin 40 in one or multiple passes. Upon conclusion of the procedure, operation of the air pump 17 may be terminated and the suction device 20 removed from the skin 40. Movement of the suction device 20 in the gliding motion over the skin 40 may facilitate migration of the adipose cells 45 into the subdermal regions of the skin 40 to enhance or improve and smoothen the contour of the skin 40, as illustrated in FIG. 13, as well as improve projection in desired areas of the patient's body.

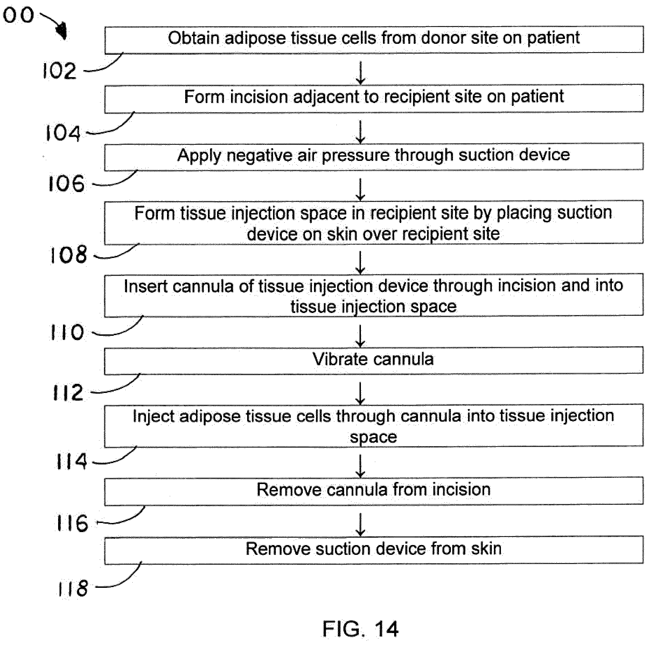

Referring next to FIG. 14 of the drawings, a flow diagram 100 of an illustrative embodiment of the external and internal expansion vibration lipofilling methods in shaping the body surface of a patient is illustrated. The method may include obtaining or harvesting adipose cells from a donor site on a patient at Step 102.

At Step 104, an incision may be formed in the skin of the patient adjacent to a recipient injection site.

At Step 106, negative pressure may be applied through a suction device.

At Step 108, a tissue injection space may be formed in the skin at the recipient injection site by placing the suction device on the skin over the recipient injection site.

At Step 110, a cannula of a tissue injection device may be inserted through the incision and into the tissue injection space.

At Step 112, the cannula may be vibrated or reciprocated.

At Step 114, the harvested adipose cells may be injected through the cannula into the tissue injection space.

At Step 116, the cannula may be removed from the incision.

At Step 118, the suction device may be removed from the patient's skin at the conclusion of the procedure.

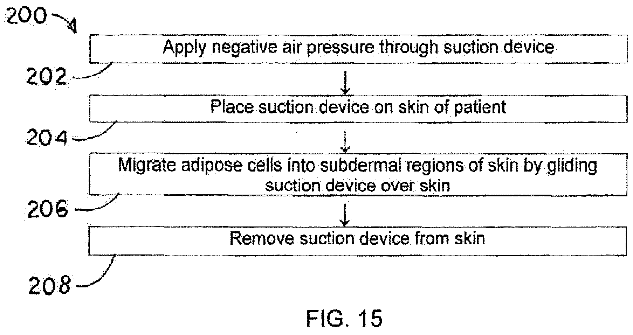

Referring next to FIG. 15 of the drawings, a flow diagram 200 of a typical method of facilitating migration of the adipose cells into subdermal regions of skin after injection of the adipose cells into the skin is illustrated.

At Step 202, negative pressure may be applied through a suction device.

At Step 204, the suction device may be placed on the skin of the patient.

At Step 206, adipose tissue cells which were previously injected into the patient may be migrated into subdermal regions of the skin by moving the suction device in a gliding motion over the skin. The suction device may be moved over the skin in one or multiple passes to achieve the desired aesthetic result.

At Step 208, the suction device may be removed from the skin at the conclusion of the procedure.

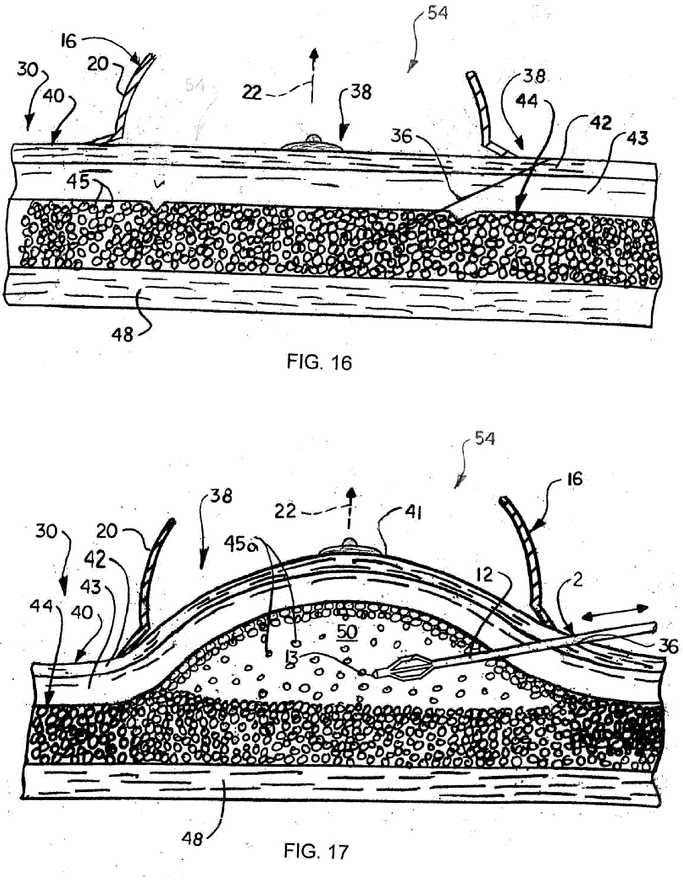

Referring next to FIGS. 16-18 of the drawings, a female breast 54 is illustrated in typical implementation of the external and internal vibration lipofilling methods in a breast augmentation procedure. Accordingly, the recipient injection site 38 may correspond to the location or position of the breast 54. After donor tumescence, a sufficient volume of adipose cells 45a which will subsequently be injected into the skin 40 at the recipient injection site 38 may initially be harvested from at least one harvest or donor site (not illustrated) on the patient 30 typically by operation of the tissue injection device 2 (FIG. 1). The pump 9 of the tissue injection device 2 may be operated in the suction phase to draw the harvested adipose cells 45a from the donor site through the cannula 12, gripping portion 3 and tubing 14, respectively, into the container 10. The cannula 12 may then be withdrawn from the donor site.

As illustrated in FIG. 16, as the patient 30 typically lies in a supine or lateral position, an incision 36 may be formed in the skin 40 adjacent to the recipient injection site 38 which corresponds to the location of the breast 54. The incision 36 may extend through the epidermis 42 and the dermis 43 and into the underlying adipose tissue layer 44 beneath the breast 54.

As illustrated in FIG. 17, the suction device 20 on the medical suction apparatus 16 of the system 1 (FIG. 1) may be placed against the surface of the skin 40 over the recipient injection site 38 and the air pump 17 operated to externally apply negative pressure 22 to the surface of the skin 40 through the tubing 24 and the interior of the suction device 20. The negative pressure 22 may form a raised or convex suctioned skin area 41 on the surface of the skin 40, and a tissue injection space 50 may be formed in the adipose tissue layer 44 beneath the suctioned skin area 41.

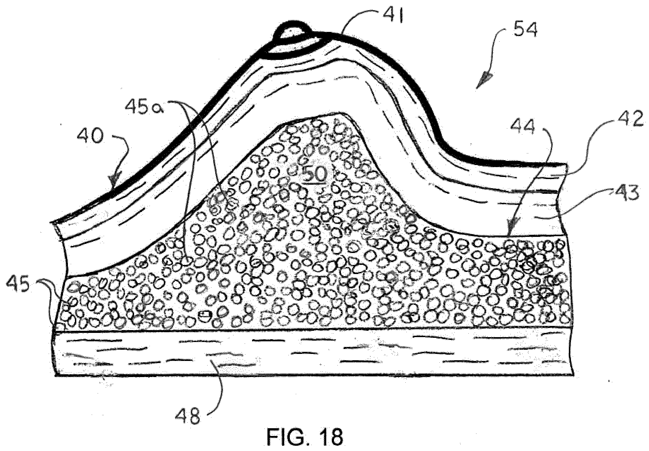

As further illustrated in FIG. 17, the cannula 12 may be inserted through the incision 36 until the cannula tip 13 of the cannula 12 terminates in the tissue injection space 50 as the suction device 20 is maintained in place on the skin 40,. The pump 9 (FIG. 1) of the tissue injection device 2 may be operated in the injection phase to pump the harvested adipose cells 45a from the container 10 through the tubing 14, gripping portion 2 and cannula 12, respectively, and into the tissue injection site 50. A typical flow rate of the adipose cells 45a is 200-300 cc/min. In some applications, the vibratory motor 4 (FIG. 1) of the tissue injection device 2 may simultaneously be operated to vibrate or reciprocate the injection cannula 12 and achieve enhanced dispersal and homogenous distribution of the harvested adipose cells 45a throughout the tissue injection site 50. In some applications, the tissue injection device 2 may be repeatedly moved in a forward-reverse action to facilitate controlled dispersal of the adipose cells 45a throughout the tissue injection space 50. The adipose cells 45a may be injected in different areas of the recipient injection site 38 to shape the breast 54 and achieve the desired aesthetic outcome, as illustrated in FIG. 18.

While certain illustrative embodiments of the disclosure have been described above, it will be recognized and understood that various modifications can be made to the embodiments and the appended claims are intended to cover all such modifications which may fall within the spirit and scope of the disclosure.

## Claims

1. An external and internal expansion vibration lipofilling system (1) for shaping the body surface of a patient (30), comprising:
a medical suction apparatus (16) having an air pump (17) and a suction device (20) disposed in pneumatic communication with the air pump (17); and
a tissue injection device (2) configured to be used in conjunction with the medical suction apparatus (16), the tissue injection device (2) including:
a gripping portion (3);
a cannula (12) disposed in fluid communication with the gripping portion (3);
a pump (9) disposed in fluid communication with the cannula (12); and
a container (10) disposed in fluid communication with the pump (9);
the suction device (20) of the medical suction apparatus (16) configured to induce formation of a tissue injection space (50) in the skin (40) of the patient (30)by applying negative pressure to the skin (40) of the patient (30); and
the tissue injection device (2) operable to simultaneously inject adipose cells from the container (10) through the gripping portion (3) and the cannula (12), respectively, into the tissue injection space (50).

2. The system of claim 1 further comprising a vibratory motor (4) engaging the gripping portion (3) for vibration, preferably wherein the vibratory motor (4) is electric, hydraulic, pneumatic or any combination thereof.

3. An external and internal expansion vibration lipofilling method for shaping the body surface of a patient (30), comprising:
modifying the body surface of the patient (30) by:
forming a tissue injection space (50) in the skin (40) of the patient (30) by externally applying negative pressure to the skin (40) of the patient (30); and
simultaneously injecting adipose cells into the tissue injection space (50).

4. The method of claim 3 further comprising vibrating the adipose cells simultaneous with injecting the adipose cells into the tissue injection space (50).

5. The method of claim 4 wherein vibrating the adipose cells comprises vibrating the adipose cells with a frequency within the range of about 2,000-10,000 cycles/min, preferably with a frequency of about 7,000 cycles/min.

6. The method of any of claims 3 to 5 further comprising forming an incision (36) in the skin (40), and wherein simultaneously injecting the adipose cells into the tissue injection space (50) comprises simultaneously injecting the adipose cells into the tissue injection space (50) through the incision (36).

7. The method of any of claims 3 to 6 wherein forming a tissue injection space (50) in the skin (40) of the patient (30) by externally applying negative pressure to the skin (40) of the patient (30) comprises forming a raised or convex suctioned skin area (41) on the surface of the skin (40) over the tissue injection space (50).

8. The method of any of claims 3 to 7 wherein modifying the body surface of the patient (30) comprises eliminating at least one skin crease (46) in the skin (40) or comprises augmenting a breast (54) on the patient (30).

9. The method of any of claims 3 to 8 further comprising migrating the adipose cells into subdermal regions of the skin (40).

10. An external and internal expansion vibration lipofilling method for shaping the body surface of a patient (30), comprising:
modifying the body surface of the patient (30) by:
obtaining an external and internal expansion vibration lipofilling system (1) including a tissue injection device (2) having a cannula (12) with a cannula tip (13) and a medical suction apparatus (16) having a suction device (20);
forming an incision (36) in the skin (40) adjacent to a recipient injection site (36);
placing the suction device (20) of the medical suction apparatus (16) against the surface of the skin (40) over the recipient injection site (38) to achieve an airtight seal between the suction device (20) and the skin (40);
forming a tissue injection space (50) in the skin (40) of the patient (30) at the recipient injection site (38) by externally applying negative pressure to the skin (40) of the patient (30) through the suction device (20) at the recipient injection site (38);
inserting the cannula (12) of the tissue injection device (2) through the incision (36) until the cannula tip (13) of the cannula (12) terminates in the tissue injection space (50); and
injecting adipose cells through the cannula (12) into the tissue injection space (50) while maintaining the suction device (20) in place on the skin (40).

11. The method of claim 10 further comprising vibrating the cannula (12) of the tissue injection device (2) simultaneous with injecting the adipose cells into the tissue injection space (50).

12. The method of claim 11 wherein vibrating the cannula (12) of the tissue injection device (2) comprises vibrating the cannula (12) of the tissue injection device (2) with a frequency within the range of about 2,000-10,000 cycles/min, preferably with a frequency of about 7,000 cycles/min.

13. The method of any of claims 10 to 12 wherein forming the tissue injection space (50) in the skin (40) of the patient (30) at the recipient injection site (38) by externally applying negative pressure to the skin (40) of the patient (30) through the suction device (20) at the recipient injection site (38) comprises forming a raised or convex suctioned skin area on the surface of the skin (40) over the tissue injection space (50).

14. The method of any of claims 10 to 13 wherein modifying the body surface of the patient (30) comprises eliminating at least one skin crease (46) in the skin (40), or comprises augmenting a breast (54) on the patient (30).

15. The method of claim 10 further comprising migrating the adipose cells into subdermal regions of the skin (40) by gliding the suction device (20) on the medical suction apparatus (16) over the skin (40).
